# EUROPEAN PATENT APPLICATION

(11) **EP 2 762 190 A1**
(43) Date of publication of application: **06.08.2014**
(21) Application number: 14152815.8
(22) Date of filing: 28.01.2014
(51) Int. Cl.: A61M 25/09, A61M 25/00, A61M 25/01

(54) **Slitted pipe and guidewire including the same**

(30) Priority: 31.01.2013 JP 2013016964
(71) Applicant: ASAHI INTECC CO., LTD., Nagoya-shi, Aichi 463-0024 (JP)
(72) Inventor: Katsuno, Masatoshi, Nagoya-shi, Aichi 463-0024 (JP)
(74) Representative: Winter, Brandl, Fürniss, Hübner, Röss, Kaiser, Polte - Partnerschaft

(57) **Abstract**

A slitted pipe includes a plurality of parallel slit patterns (15a to 15f) provided in an area except for both end portions in a longitudinal direction to extend in the longitudinal direction in a helical shape.

## Description

### BACKGROUND OF THE INVENTION

### 1. Field of the Invention

The present invention relates to a slitted pipe having slits in a surface, and a guidewire including the slitted pipe.

### 2. Description of the Related Art

Various guidewires have hitherto been proposed to guide a medical instrument, for example, a catheter to be inserted in a tubular organ such as a blood vessel, a digestive tract, or a ureter, or a body tissue.

For example, U.S. Patent No. 5,345,945 (Patent Document 1) describes that an inner coil is fixed to a distal end of a guidewire by a brazing part (see Fig. 1B of Patent Document 1). Patent Document 1 also describes that, when a distal end of an inner coil is located apart from a distal end of a guidewire toward a proximal end, the inner coil is fixed to a brazing part (see Figs. 5A and 5B of Patent Document 1).

In contrast, Japanese Unexamined Patent Application Publication (Translation of PCT Application) No. 2007-514458 (Patent Document 2) describes a reinforcing member (tubular member) having a plurality of cuts formed by grooves or one helical cut (see Figs. 3A and 3B of Patent Document 2).

In the guidewire described in Patent Document 1, end portions of a wire that forms the inner coil may get loose. Hence, when the guidewire is produced, it is necessary to braze the end portions of the inner coil to a core wire so that the end portions will not get loose. This causes troublesomeness in production.

Since the inner coil is helically wound around the core wire, when the end portions of the inner coil are brazed, the inner coil needs to be brazed over the entire circumferential cross section. The inner coil is brazed to the core wire also with attention to this point.

In the reinforcing member illustrated in Fig. 3B of Patent Document 2, since a plurality of grooves are concentrically formed in the tubular member, rigidity (especially, flexibility) of the reinforcing member can be adjusted. However, it is difficult to expect enhancement of torque transmissibility to a distal end of a guidewire when the manipulator rotates a proximal end of the guidewire and an increase in thrust force of the guidewire in a lesion.

Further, in the reinforcing member illustrated in Fig. 3A of Patent Document 2, since only one helical cut is provided in the tubular member, a section other than the cut is formed by one strip. Hence, flexibility of the tubular member formed by one strip is insufficient. Further, if this one strip is cut off in the body, there is a safety problem during use of the guidewire, that is, the guidewire itself cannot be removed from the body of the patient.

Also, since only one helical cut is provided in the tubular member in this reinforcing member, the section other than the cut has a structure similar to that of a spring formed by a single wire, and has a structural deviation in the entire cross section of the tubular member. Hence, it is also necessary to remove the deviation to enhance well-balanced torque transmissibility.

In a sense, the cut is a factor for formation of play when the guidewire is rotated. Hence, it is necessary to set the width of the cut in consideration of the play of the guidewire and to minimize expansion and contraction of the tubular member in an axial direction.

For example, when the guidewire enters a chronic total occlusion lesion (hereinafter referred to as a CTO lesion), the distal end of the guidewire does sometimes not advance into the CTO lesion even when the manipulator repeats operations of pushing and pulling the proximal end of the guidewire. Since it is extremely important to enhance torque transmissibility of the guidewire in the CTO lesion, measures have recently been tried to further advance the distal end of the guidewire into the CTO lesion by enhancing torque transmissibility in rotation of the guidewire in at least one direction.

### SUMMARY OF THE INVENTION

The present invention has been made in view of the above circumstances, and an object of the invention is to provide a slitted pipe that enhances torque transmissibility in rotation in at least one direction and improves safety when the slitted pipe is cut off, a slitted pipe that enhances torque transmissibility in a well-balanced manner in a circumferential cross section, or a slitted pipe that prevents expansion and contraction in an axial direction, and a guidewire that uses the slitted pipe and that can be produced easily.

A slitted pipe according to a first aspect includes a plurality of parallel slit patterns provided in an area except for both end portions in a longitudinal direction to extend in the longitudinal direction in a helical shape.

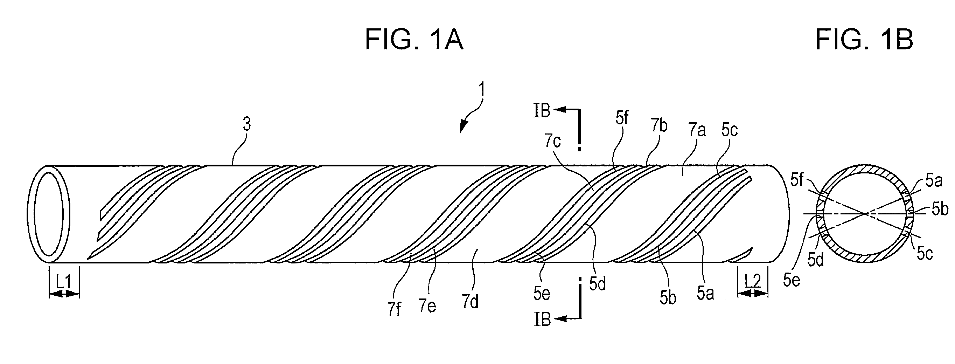

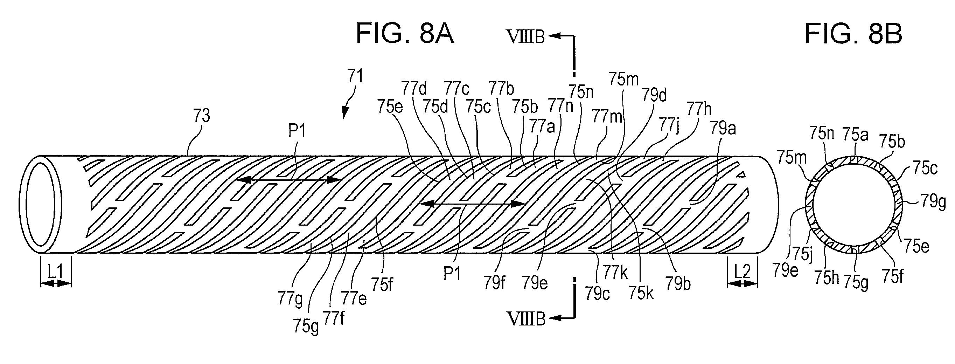

The slit pattern can comprise only one slit (Fig. 1A to 7A) or a plurality of slits divided by the girders and arranged on the same helical shape in a row (Fig. 8A to 10A).

According to the slitted pipe of the first aspect, the slitted pipe itself can be made flexible, and the end portions of the slitted pipe are prevented from getting loose. Moreover, torque transmissibility in rotation in at least one rotation can be enhanced, and safety of the slitted pipe when the slitted pipe is cut off can be improved.

A guidewire according to a second aspect of the present invention includes a core shaft, and the above slitted pipe covering a distal end portion of the core shaft.

According to the guidewire of the second aspect, the guidewire can be easily produced, and torque transmissibility of the guidewire in rotation in at least one direction can be enhanced. Moreover, safety of the guidewire when the guidewire is cut off can be improved.

A guidewire according to a third aspect of the present invention includes a core shaft, and the above slitted pipe covering a distal end portion of the core shaft such that the other end of the slitted pipe is located on a distal side of the core shaft.

According to the guidewire of the third aspect, the guidewire can be easily produced, torque transmissibility in rotation in at least one direction of the guidewire can be enhanced, and safety of the guidewire when the guidewire is cut off can be improved. Moreover, flexibility of the distal end portion of the guidewire can be further enhanced by the slitted pipe.

A guidewire according to a fourth aspect of the present invention includes a core shaft, a coil body covering a distal end portion of the core shaft, and the above slitted pipe disposed in the coil body to cover the distal end portion of the core shaft.

According to the guidewire of the fourth aspect, the guidewire can be easily produced, torque transmissibility of the guidewire in rotation in at least one direction can be enhanced, and safety of the guidewire when the guidewire is cut off can be improved. Moreover, flexibility of the guidewire can be adjusted by the slitted pipe disposed therein while the outer diameter of the coil body is maintained.

A guidewire according to a fifth aspect includes a core shaft, a coil body covering a distal end portion of the core shaft, and the above slitted pipe disposed in the coil body to cover the distal end portion of the core shaft such that the other end of the slitted pipe is located on a distal side of the core shaft.

According to the guidewire of the fifth aspect, the guidewire can be easily produced, torque transmissibility of the guidewire in rotation in at least one direction can be enhanced, and safety of the guidewire when the guidewire is cut off can be improved. Moreover, flexibility of the distal end portion of the guidewire can be adjusted by the slitted pipe disposed therein while the outer diameter of the coil body is maintained, and flexibility of the distal end portion of the guidewire can be enhanced.

### BRIEF DESCRIPTION OF THE DRAWINGS

Fig. 1A is an overall perspective view of a slitted pipe according to a first embodiment of the present invention, and Fig. 1B is a cross-sectional view taken along line IB-IB of Fig. 1A;
Fig. 2A is an overall perspective view of a slitted pipe according to a second embodiment of the present invention, and Fig. 2B is a cross-sectional view taken along line IIB-IIB of Fig. 2A;
Fig. 3A is an overall perspective view of a slitted pipe according to a third embodiment of the present invention, and Fig. 3B is a cross-sectional view taken along line IIIB-IIIB of Fig. 3A;
Fig. 4A is an overall perspective view of a slitted pipe according to a fourth embodiment of the present invention, Fig. 4B is a cross-sectional view taken along line IVB-IVB of Fig. 4A, Fig. 4C is a cross-sectional view taken along line IVC-IVC of Fig. 4A, and Fig. 4D is a cross-sectional view taken along line IVD-IVD of Fig. 4A;
Fig. 5A is an overall perspective view of a slitted pipe according to a fifth embodiment of the present invention, Fig. 5B is a cross-sectional view taken along line VB-VB of Fig. 5A, Fig. 5C is a cross-sectional view taken along line VC-VC of Fig. 5A, and Fig. 5D is a cross-sectional view taken along line VD-VD of Fig. 5A;
Fig. 6A is an overall perspective view of a slitted pipe according to a sixth embodiment of the present invention, Fig. 6B is a cross-sectional view taken along line VIB-VIB of Fig. 6A, Fig. 6C is a cross-sectional view taken along line VIC-VIC of Fig. 6A, and Fig. 6D is a cross-sectional view taken along line VID-VID of Fig. 6A;
Fig. 7A is an overall perspective view of a slitted pipe according to a seventh embodiment of the present invention, Fig. 7B is a cross-sectional view taken along line VIIB-VIIB of Fig. 7A, Fig. 7C is a cross-sectional view taken along line VIIC-VIIC of Fig. 7A, and Fig. 7D is a cross-sectional view taken along line VIID-VIID of Fig. 7A;
Fig. 8A is an overall perspective view of a slitted pipe according to an eighth embodiment of the present invention, and Fig. 8B is a cross-sectional view taken along VIIIB-VIIIB of Fig. 8A;
Fig. 9A is an overall perspective view of a slitted pipe according to a ninth embodiment of the present invention, and Fig. 9B is a cross-sectional view taken along line IXB-IXB of Fig. 9A;
Fig. 10A is an overall perspective view of a slitted pipe according to a tenth embodiment of the present invention, and Fig. 10B is a cross-sectional view taken along line XB-XB of Fig. 10A;
Fig. 11 is an overall view of a guidewire according to an eleventh embodiment of the present invention;
Fig. 12 is an overall view of a guidewire according to a twelfth embodiment of the present invention; and
Fig. 13 is an overall view of a guidewire according to a thirteenth embodiment of the present invention.

### DESCRIPTION OF THE PREFERRED EMBODIMENTS

First, a slitted pipe according to the present invention will be described on the basis of preferred embodiments illustrated in the drawings.

### First Embodiment

Fig. 1A is an overall perspective view of a slitted pipe according to a first embodiment of the present invention, and Fig. 1B is a cross-sectional view taken along line IB-IB of Fig. 1A.

In Figs. 1A and 1B and Figs. 2A to 10B described below, for easy understanding, the slitted pipe is schematically illustrated as a whole while being shortened in a longitudinal direction. Hence, overall dimensions of the slitted pipe are different from actual ones.

Referring to Figs. 1A and 1B, in a slitted pipe 1 of the first embodiment, three parallel slit patterns 5a, 5b, and 5c extending in a helical shape in a longitudinal direction of a tubular member 3 are formed in a surface of the tubular member 3 by laser machining or etching such that the slit patterns 5a, 5b, and 5c penetrate the tubular member 3.

In the first embodiment, three slit patterns 5d, 5e, and 5f are also provided at an opposite position of the tubular member 3 in cross section (a position apart 180 degrees). That is, the helical slit patterns 5a to 5c and the helical slit patterns 5d to 5f are disposed at positions point-symmetrical to each other with respect to the center point of the cross section of the slitted pipe 1. More specifically, the slit pattern5d is disposed at a position point-symmetrical to the slit pattern 5a, the slit pattern 5e is disposed at a position point-symmetrical to the slit pattern 5b, and the slit pattern 5f is disposed at a position point-symmetrical to the slit pattern 5c.

The helical slit patterns do not always need to be disposed at positions point-symmetrical with respect to the center point of the cross section of the slitted pipe 1, and for example, the object of the present invention is achieved by adopting only the helical slit patterns 5a to 5c. However, when a plurality of helical slit patterns are arranged at the positions point-symmetrical with respect to the center point of the cross section of the slitted pipe 1, torque transmissibility of the slitted pipe itself can be enhanced in a well-balanced manner in a circumferential cross section.

In contrast, slit patterns are not formed in both longitudinal end portions of the slitted pipe 1. The slit patterns 5a to 5f are not formed within a distance L1 from a left end of the slitted pipe 1 and a distance L2 from a right end of the slitted pipe 1 in Fig. 1A. That is, the slit patterns 5a to 5f form a closed space in the slitted pipe 1. Therefore, the slitted pipe 1 is not divided by the slit patterns 5a to 5f, but can be handled as one piece. This allows the slitted pipe 1 to be handled easily.

Belt-like helical portions 7a to 7f defined by the slit patterns 5a to 5f can independently operate. Even if any of the helical portions 7a to 7f is cut off, the remaining helical portions can prevent the slitted pipe 1 from being cut off completely. Therefore, while the six slit patterns 5a to 5f are formed in the first embodiment, as long as the slitted pipe has at least two slit patterns, it can be prevented from completely cut off even if any of the slit patterns is cut off.

Since the slitted pipe 1 has the slit patterns 5a to 5f in its surface, flexibility thereof can be enhanced. Hence, when the slitted pipe 1 is inserted in a blood vessel of a patient, perforation of the blood vessel can be prevented.

Since the slitted pipe 1 has the helical slit patterns 5a to 5f in its surface, play is formed when rotational force applied to one end of the slitted pipe 1 is transmitted to the other end. Hence, even if great force is suddenly applied to one end of the slitted pipe 1, the force can be prevented from being immediately transmitted to the other end. Thus, an accident can be avoided.

Since the slitted pipe 1 has the helical slit patterns 5a to 5f in its surface, even if one end of the slitted pipe 1 is locked for any reason, torque transmissibility of the slitted pipe 1 to the other end can be enhanced by further rotating the slitted pipe 1. This increases the possibility that the slitted pipe 1 will be unlocked.

In the first embodiment, the slit patterns 5a to 5f extend in a left-handed helical shape along the length of the tubular member 3. Hence, torque transmissibility to the other end of the slitted pipe 1 can be enhanced by rotating one end of the slitted pipe 1 in a direction to narrow the slits of the slit patterns 5a to 5f, that is, in a rightward direction.

While the material of the slitted pipe 1 is not particularly limited, stainless steel (SUS304) is used in the first embodiment. Alternatively, a superelastic alloy, such as a Ni-Ti alloy, can be used.

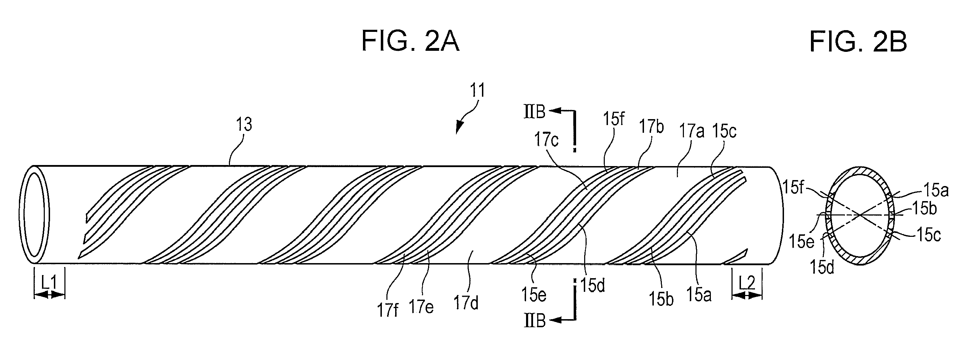

While the slitted pipe 1 of the first embodiment is shaped like a hollow cylinder, it may have other shapes. For example, the slitted pipe 1 may be shaped like a prism that is polygonal, such as triangular, quadrangular, hexagonal, or octagonal, in cross section. Alternatively, the slitted pipe 1 may be elliptical in cross section, as illustrated in Fig. 2B.

However, as described above, when the slitted pipe 1 is shaped like a hollow cylinder as in the first embodiment, torque transmissibility to the other end of the slitted pipe 1 when rotational force is applied to one end can be further enhanced because of the relationship with a plurality of helical slit patterns provided in the surface of the tubular member.

### Second Embodiment

Fig. 2A is an overall perspective view of a slitted pipe according to a second embodiment of the present invention, and Fig. 2B is a cross-sectional view taken along line IIB-IIB of Fig. 2A.

Referring to Figs. 2A and 2B, a slitted pipe 11 according to the second embodiment is similar to the slitted pipe 1 of the above-described first embodiment except that the cross section of the slitted pipe 11 is elliptical. In the slitted pipe 11, six parallel and helical slit patterns 15a, 15b, 15c, 15d, 15e, and 15f extending in a longitudinal direction of a tubular member 13 are provided in a surface of the tubular member 13 such as to penetrate the tubular member 13.

The slitted pipe 11 can be used when it is to be curved in one direction, but is not to be curved in the other directions. For example, in a case in which the slitted pipe 11 is used in a two-dimensional space, a peculiar effect is achieved when the slitted pipe 11 is to be curved only in a direction of the short side of the elliptical cross section, but is not to be curved in a direction of the long side of the elliptical cross section.

In both longitudinal end portions of the slitted pipe 11, slit patterns are not formed, similarly to the above-described slitted pipe 1. That is, the slit patterns 15a to 15f are not formed within a distance L1 from a left end of the slitted pipe 11 and a distance L2 from a right end of the slitted pipe 11. Therefore, similarly to the slitted pipe 1, the slitted pipe 11 is not divided by the slit patterns 15a to 15f, but can be handled as one piece. This allows the slitted pipe 11 to be handled easily.

The slitted pipe 11 is similar to the slitted pipe 1 in the following respects. Since belt-like helical portions 17a to 17f defined by the slit patterns 15a to 15f can independently operate, even if any of the slit patterns 15a to 15f is cut off, the slitted pipe can be prevented from being cut off completely. Moreover, since the slitted pipe 11 has the helical slit patterns 15a to 15f in its surface, play is formed when rotational force applied to one end of the slitted pipe 11 is transmitted to the other end. Hence, even if great force is suddenly applied to one end of the slitted pipe 11, it is prevented from being immediately transmitted to the other end. Further, since the slitted pipe 11 has the helical slit patterns 15a to 15f in its surface, even if one end of the slitted pipe 11 is locked for any reason, torque transmissibility to the other end of the slitted pipe 11 can be enhanced by further rotating the slitted pipe 11.

### Third Embodiment

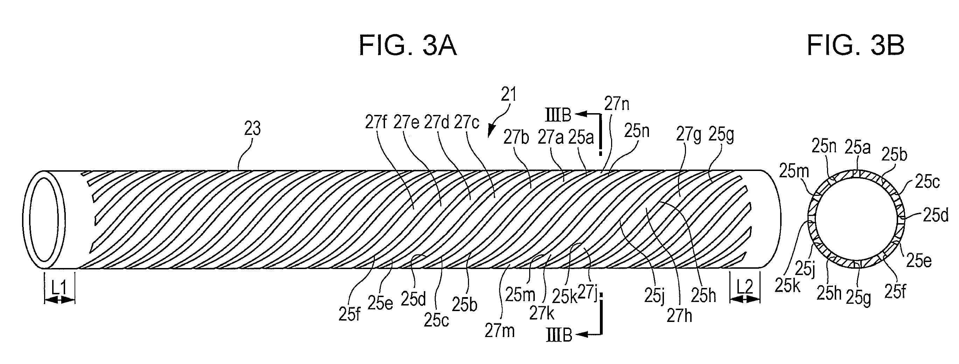

Fig. 3A is an overall perspective view of a slitted pipe according to a third embodiment of the present invention, and Fig. 3B is a cross-sectional view taken along line IIIB-IIIB of Fig. 3A.

Referring to Figs. 3A and 3B, a slitted pipe 21 according to the third embodiment is different from the first and second embodiments in that twelve parallel and helical slit patterns 25a, 25b, 25c, 25d, 25e, 25f, 25g, 25h, 25j, 25k, 25m, and 25n extending in a longitudinal direction of a tubular member 23 are equally spaced in the entire cross section of the slitted pipe 21. According to this slitted pipe 21, since the helical slit patterns are equally spaced in the entire cross section, torque transmissibility of the slitted pipe itself can be enhanced in a well-balanced manner in the circumferential cross section.

In the third embodiment, the helical slit patterns are also located at positions point-symmetrical with respect to the center point of the cross section of the slitted pipe 21.

Similarly to the slitted pipe 1 and the slitted pipe 11 described above, slit patterns are not formed in both longitudinal end portions of the slitted pipe 21. That is, the slit patterns 25a to 25n are not formed within a distance L1 from a left end and a distance L2 from a right end of the slitted pipe 21 in Fig. 3A. Therefore, the slitted pipe 21 is not divided by the slit patterns 25a to 25n, but can be handled as one piece. This allows the slitted pipe 21 to be handled easily.

Since belt-like helical portions 27a to 27n defined by the slit patterns 25a to 25n can independently operate, for example, even if any of the helical portions 27a to 27n is cut off, the remaining helical portions can prevent the slitted pipe 21 from being completely cut off.

Since the slitted pipe 21 has the slit patterns 25a to 25n in its surface, flexibility thereof can be enhanced further. When the slitted pipe 21 is inserted in a blood vessel of a patient, perforation of the blood vessel can be prevented further.

Since the slitted pipe 21 has the helical slit patterns 25a to 25n in its surface, much play is formed when rotational force applied to one end of the slitted pipe 21 is transmitted to the other end. Hence, even if great force is suddenly applied to one end of the slitted pipe 21, the force can be prevented from being immediately transmitted to the other end. Thus, an accident can be further avoided.

Since the slitted pipe 21 has the helical slit patterns 25a to 25n in its surface, even if one end of the slitted pipe 21 is locked for any reason, torque transmissibility to the other end of the slitted pipe 21 can be enhanced by further rotating the slitted pipe 21, similarly to the slitted pipe 1 and the slitted pipe 11.

### Fourth Embodiment

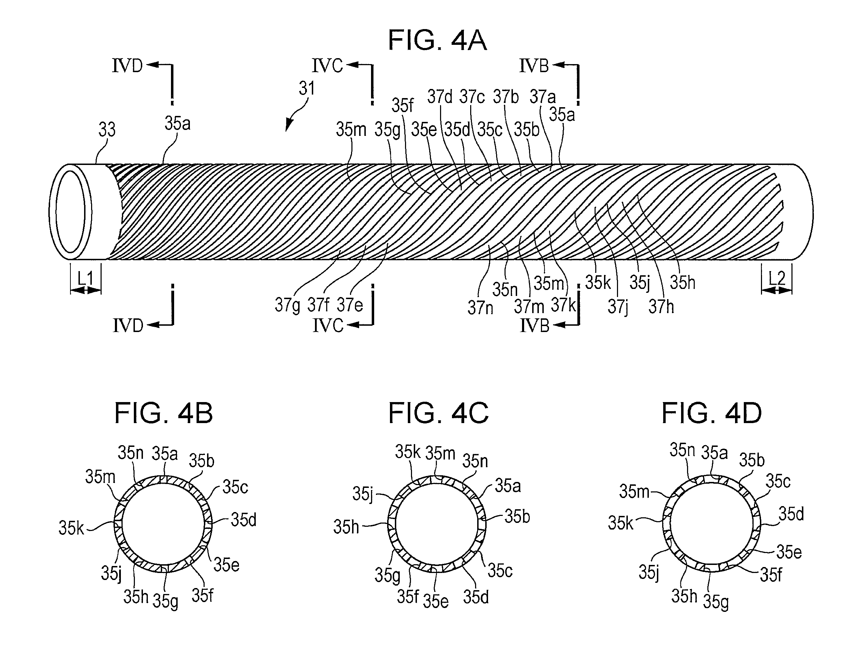

Fig. 4A is an overall perspective view of a slitted pipe according to a fourth embodiment of the present invention, Fig. 4B is a cross-sectional view taken along line IVB-IVB of Fig. 4A, Fig. 4C is a cross-sectional view taken along line IVC-IVC of Fig. 4A, and Fig. 4D is a cross-sectional view taken along line IVD-IVD of Fig. 4A.

Referring to Figs. 4A to 4D, a slitted pipe 31 of the fourth embodiment is different from the above-described first to third embodiments in that the pitch of twelve parallel and helical slit patterns extending in a longitudinal direction of a tubular member 33 gradually decreases from one end to the other end of the slitted pipe 31. More specifically, the pitch of helical slit patterns 35a, 35b, 35c, 35d, 35e, 35f, 35g, 35h, 35j, 35k, 35m, and 35n gradually decreases from one end (a right end in Fig. 4A) toward the other end (a left end in Fig. 4A) of the slitted pipe 31. According to this slitted pipe 31, flexibility at the other end (left end in Fig. 4A) of the slitted pipe 31 can be enhanced. Therefore, when the slitted pipe 31 is inserted in a blood vessel of a patient, perforation of the blood vessel can be prevented further.

In the fourth embodiment, the helical slit patterns are also located at positions point-symmetrical with respect to the center point of the cross section of the slitted pipe 31, and slit patterns are not formed in both longitudinal end portions of the slitted pipe 31. That is, the slit patterns 35a to 35n are not formed within a distance L1 from the left end of the slitted pipe 31 and a distance L2 from the right end of the slitted pipe 31 in Fig. 4A. Therefore, the slitted pipe 31 is not divided by the slit patterns 35a to 35n, but can be handled as one piece. This allows the slitted pipe 31 to be handled easily.

Since belt-like helical portions 37a to 37n defined by the slit patterns 35a to 35n can independently operate, for example, even if any of the helical portions 37a to 37n is cut off, the remaining helical portions can prevent the slitted pipe 31 from being cut off completely.

The slitted pipe 31 is similar to the slitted pipe 1, the slitted pipe 11, and the slitted pipe 21 in that, even if great force is suddenly applied to one end of the slitted pipe 31, it can be further prevented from being immediately transmitted to the other end and in that torque transmissibility to the other end of the slitted pipe 31 can be enhanced by further rotating the slitted pipe 31.

### Fifth Embodiment

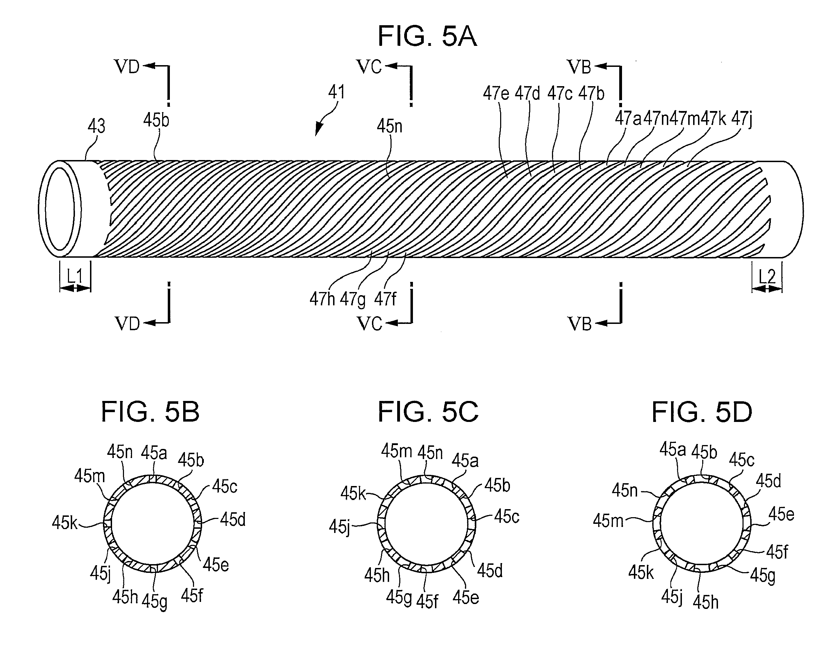

Fig. 5A is an overall perspective view of a slitted pipe according to a fifth embodiment of the present invention, Fig. 5B is a cross-sectional view taken along line VB-VB of Fig. 5A, Fig. 5C is a cross-sectional view taken along line VC-VC of Fig. 5A, and Fig. 5D is a cross-sectional view taken along line VD-VD of Fig. 5A.

Referring to Figs. 5A to 5D, a slitted pipe 41 of the fifth embodiment is different from the above-described first to fourth embodiments in that the pitch of twelve parallel and helical slit patterns extending in a longitudinal direction of a tubular member 43 decreases in a stepwise manner from one end toward the other end. More specifically, the pitch of helical slit patterns 45a, 45b, 45c, 45d, 45e, 45f, 45g, 45h, 45j, 45k, 45m, and 45n decreases in three steps from one end (a right end in Fig. 5A) toward the other end (a left end in Fig. 5A) of the slitted pipe 41. According to this slitted pipe 41, flexibility at the other end (left end) of the slitted pipe 41 can be enhanced. Hence, when the slitted pipe 41 is inserted in a blood vessel of a patient, perforation of the blood vessel can be further prevented, and the base point can be easily formed when the slitted pipe 41 is curved.

In the fifth embodiment, the slit patterns 45a to 45n are also not formed within a distance L1 from the left end of the slitted pipe 41 and a distance L2 from the right end of the slitted pipe 41. Hence, the slitted pipe 41 is not divided by the slit patterns 45a to 45n, but can be handled as one piece. This allows the slitted pipe 41 to be handled easily.

Since belt-like helical portions 47a to 47n defined by the slit patterns 45a to 45n can independently operate, for example, even if any of the helical portions 47a to 47n is cut off, the remaining helical portions can prevent the slitted pipe 41 from being cut off completely.

The slitted pipe 41 is similar to the slitted pipe 1, the slitted pipe 11, the slitted pipe 21, and the slitted pipe 31 in that, even if great force is suddenly applied to one end of the slitted pipe 41, it can be further prevented from being immediately transmitted to the other end and in that torque transmissibility to the other end of the slitted pipe 41 can be enhanced by further rotating the slitted pipe 41.

### Sixth Embodiment

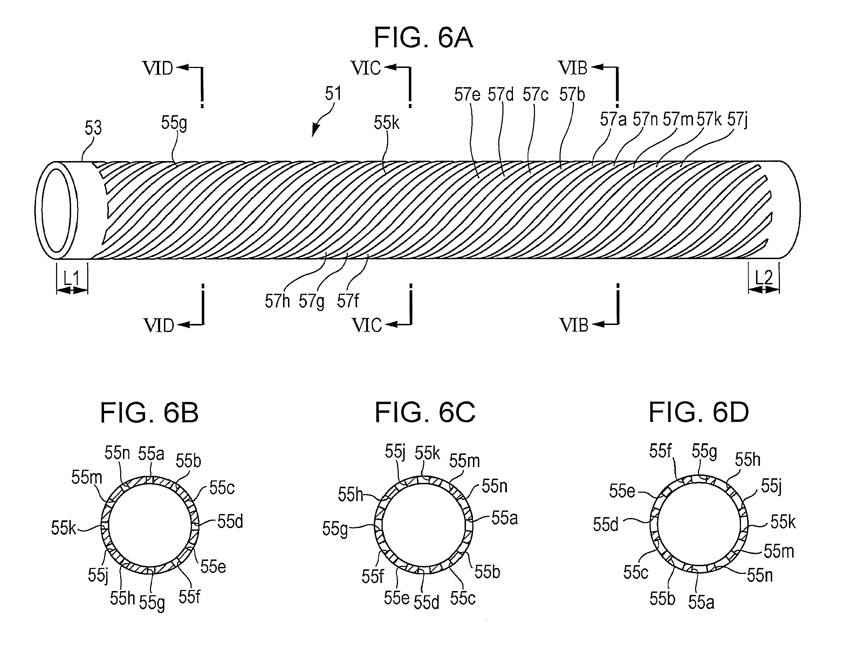

Fig. 6A is an overall perspective view of a slitted pipe according to a sixth embodiment of the present invention, Fig. 6B is a cross-sectional view taken along line VIB-VIB of Fig. 6A, Fig. 6C is a cross-sectional view taken along line VIC-VIC of Fig. 6A, and Fig. 6D is a cross-sectional view taken along line VID-VID of Fig. 6A.

Referring to Figs. 6A to 6D, a slitted pipe 51 of the sixth embodiment is different from the above-described first to fifth embodiments in that the width of twelve parallel and helical slit patterns extending in a longitudinal direction of a tubular member 53 gradually increases from one end toward the other end. More specifically, the width of helical slit patterns 55a, 55b, 55c, 55d, 55e, 55f, 55g, 55h, 55j, 55k, 55m, and 55n gradually increases from one end (a right end in Fig. 6A) toward the other end (a left end in Fig. 6A) of the slitted pipe 51. According to this slitted pipe 51, flexibility at the other end (left end) of the slitted pipe 51 can be enhanced. Hence, when the slitted pipe 51 is inserted in a blood vessel of a patient, perforation of the blood vessel can be prevented further.

In the sixth embodiment, the slit patterns 55a to 55n are also not formed within a distance L1 from the left end of the slitted pipe 51 and a distance L2 from the right end of the slitted pipe 51. Hence, the slitted pipe 51 is not divided by the slit patterns 55a to 55n, but can be handled as one piece. This allows the slitted pipe 51 to be handled easily.

Belt-like helical portions 57a to 57n defined by the slit patterns 55a to 55n can independently operate. Hence, for example, even if any of the helical portions 57a to 57n is cut off, the remaining helical portions can prevent the slitted pipe 51 from being cut off completely.

The slitted pipe 51 is similar to the slitted pipe 1, the slitted pipe 11, the slitted pipe 21, the slitted pipe 31, and the slitted pipe 41 in that, even if great force is applied to one end of the slitted pipe 51, it can be further prevented from being immediately transmitted to the other end and in that torque transmissibility to the other end of the slitted pipe 51 can be enhanced by further rotating the slitted pipe 51.

Some modifications of the slitted pipe 51 of the sixth embodiment are conceivable. For example, the structure of the above-described fourth embodiment can be added to the structure of the slitted pipe 51. That is, the pitch of the helical slit patterns 55a to 55n extending in the longitudinal direction of the tubular member 53 may gradually decrease from one end toward the other end of the slitted pipe 51.

Alternatively, the structure of the above-described fifth embodiment can be added to the structure of the slitted pipe 51. That is, the pitch of the helical slit patterns 55a to 55n extending in the longitudinal direction of the tubular member 53 may decrease in a stepwise manner from one end toward the other end of the slitted pipe 51.

### Seventh Embodiment

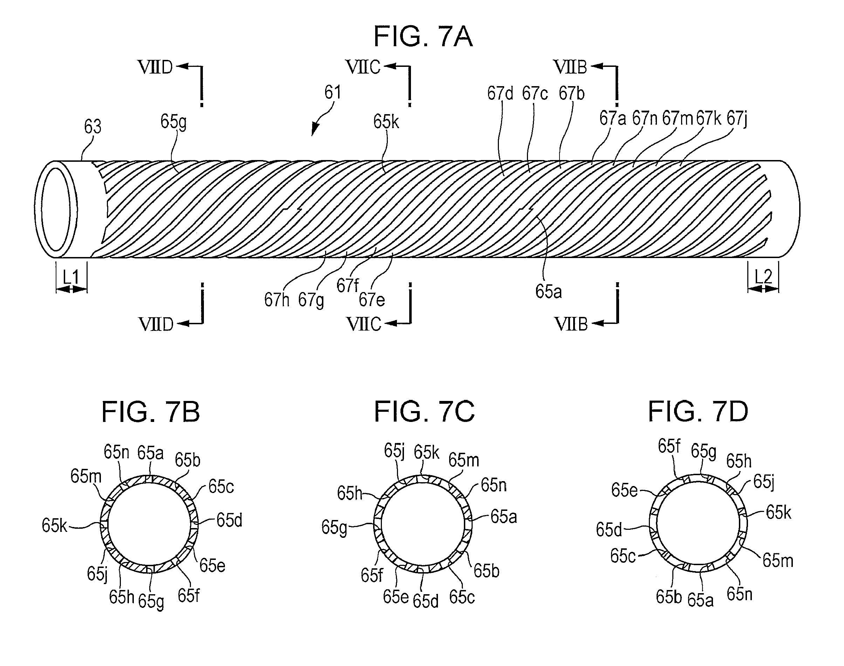

Fig. 7A is an overall perspective view of a slitted pipe according to a seventh embodiment of the present invention, Fig. 7B is a cross-sectional view taken along line VIIB-VIIB of Fig. 7A, Fig. 7C is a cross-sectional view taken along line VIIC-VIIC of Fig. 7A, and Fig. 7D is a cross-sectional view taken along line VIID-VIID of Fig. 7A.

Referring to Figs. 7A to 7D, a slitted pipe 61 of the seventh embodiment is different from the above-described first to sixth embodiments in that the width of twelve parallel and helical slit patterns extending in a longitudinal direction of a tubular member 63 increases in a stepwise manner from one end toward the other end. More specifically, the width of helical slit patterns 65a, 65b, 65c, 65d, 65e, 65f, 65g, 65h, 65j, 65k, 65m, and 65n increases in three steps from one end (a right end in Fig. 7A) toward the other end (a left end in Fig. 7A) of the slitted pipe 61. According to this slitted pipe 61, flexibility at the other end (left end) of the slitted pipe 61 can be enhanced. Hence, when the slitted pipe 61 is inserted in a blood vessel of a patient, perforation of the blood vessel can be further prevented, and the base point can be easily formed when the slitted pipe 61 is curved.

In the seventh embodiment, the slit patterns 65a to 65n are also not formed within a distance L1 from the left end of the slitted pipe 61 and a distance L2 from the right end of the slitted pipe 61. Hence, the slitted pipe 61 is not divided by the slit patterns 65a to 65n, but can be handled as one piece. This allows the slitted pipe 61 to be handled easily.

Belt-like helical portions 67a to 67n defined by the slit patterns 65a to 65n can independently operate. Hence, for example, even if any of the helical portions 67a to 67n is cut off, the remaining helical portions can prevent the slitted pipe 61 from being cut off completely.

The slitted pipe 61 is similar to the slitted pipe 1, the slitted pipe 11, the slitted pipe 21, the slitted pipe 31, the slitted pipe 41, and the slitted pipe 51 in that, even if great force is suddenly applied to one end of the slitted pipe 61, it can be further prevented from being immediately transmitted to the other end and in that torque transmissibility to the other end of the slitted pipe 61 can be enhanced by further rotating the slitted pipe 61.

Some modifications of the slitted pipe 61 of the seventh embodiment are also conceivable. For example, the structure of the above-described fourth embodiment can be added to the structure of the slitted pipe 61. That is, the pitch of the helical slit patterns 65a to 65n extending in the longitudinal direction of the tubular member 63 may gradually decrease from one end toward the other end of the slitted pipe 61.

Alternatively, the structure of the above-described fifth embodiment can be added to the structure of the slitted pipe 61. That is, the pitch of the helical slit patterns 65a to 65n extending in the longitudinal direction of the tubular member 63 may decrease in a stepwise manner from one end toward the other end of the slitted pipe 61. Eighth Embodiment

Fig. 8A is an overall perspective view of a slitted pipe according to an eighth embodiment of the present invention, and Fig. 8B is a cross-sectional view taken along line VIIIB-VIIIB of Fig. 8A.

Referring to Figs. 8A and 8B, a slitted pipe 71 of the eighth embodiment is different from the above-described first to seventh embodiments in that girders are provided on helical slit pattern to cross over the slit pattern. I.e., the girders divide the slit pattern into plurality of slits. More specifically, twelve parallel and helical slit patterns 75a, 75b, 75c, 75d, 75e, 75f, 75g, 75h, 75j, 75k, 75m, and 75n extending in a longitudinal direction of a tubular member 73 have girders 79a, 79b, 79c, 79d, 79e, 79f, 79g, ... that are disposed in the slit patterns to divide the slit patterns into a plurality of slits. As illustrated in Fig. 8B, these girders are arranged at two positions (79e and 79g) point-symmetrical with respect to the center point of the cross section. According to this slitted pipe 71, since the girders are disposed on the helical slit pattern to cross over the slit pattern, expansion and contraction of the slitted pipe 71 in an axial direction are prevented. This can further enhance torque transmissibility of the slitted pipe 71 and can further improve safety of the slitted pipe 71 when the slitted pipe 71 is cut off.

Preferably, the girders adjacent in the longitudinal direction are arranged while being shifted from each other the girders of adjacent slits by a predetermined angle in cross section per predetermined distance in the longitudinal direction. This can enhance torque transmissibility of the slitted pipe itself in a well-balanced manner in the circumferential cross section, and can further improve safety of the slitted pipe when the slitted pipe is cut off.

Preferably, the girders extend not in a direction orthogonal to the helical direction, but in a direction P1 parallel to a longitudinal axis of the tubular member 73. This can further prevent expansion and contraction of the slitted pipe 71 in the axial direction.

In the eighth embodiment, the slit patterns 75a to 75n are also not formed within a distance L1 from a left end of the slitted pipe 71 and a distance L2 from a right end of the slitted pipe 71. Hence, the slitted pipe 71 is not divided by the slit patterns 75a to 75n, but can be handled as one piece. This allows the slitted pipe 71 to be handled easily.

Belt-like helical portions 77a to 77n defined by the slit patterns 75a to 75n can independently operate. Hence, for example, even if any of the helical portions 77a to 77n is cut off, the remaining helical portions can prevent the slitted pipe from being completely cut off.

The slitted pipe 71 is similar to the slitted pipe 1, the slitted pipe 11, the slitted pipe 21, the slitted pipe 31, the slitted pipe 41, the slitted pipe 51, and the slitted pipe 61 in that, even if great force is suddenly applied to one end of the slitted pipe 71, it can be further prevented from being immediately transmitted to the other end and in that torque transmissibility to the other end of the slitted pipe 71 can be enhanced by further rotating the slitted pipe 71.

### Ninth Embodiment

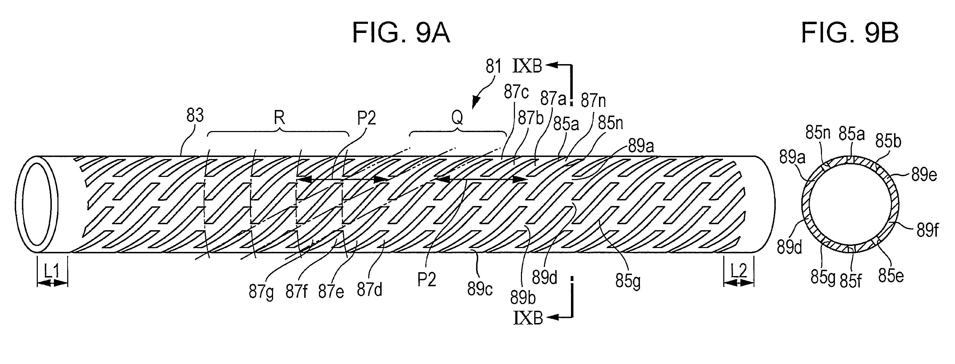

Fig. 9A is a perspective view of a slitted pipe according to a ninth embodiment of the present invention, and Fig. 9B is a cross-sectional view taken along line IXB-IXB of Fig. 9A.

Referring to Figs. 9A and 9B, in a slitted pipe 81 of the ninth embodiment, twelve parallel and helical slit patterns 85a, 85b, 85c, 85d, 85e, 85f, 85g, 85h, 85j, 85k, 85m, and 85n extending in a longitudinal direction of a tubular member 83 have girders 89a, 89b, 89c, 89d, 89e, 89f, ... that divide the slit patterns into a plurality of slits. Unlike the above-described eighth embodiment, as illustrated in Fig. 9B, these girders are arranged at four positions 89a, 89d, 89e, and 89f point-symmetrical with respect to the center point of the cross section, and form a helical shape Q or a helical shape R different from the helical shape of the slit patterns on a surface of the slitted pipe 81. According to this slitted pipe 81, torque transmissibility can be further enhanced, and safety of the slitted pipe when the slitted pipe is cut off can be further improved.

Each of the girders may be shaped such that two opposed sides (two sides crossing over the corresponding slit pattern) extend in a direction orthogonal to the helical shape, or extend in a direction P2 parallel to a longitudinal axis of the tubular member 83. That is, in a case in which two opposed sides extend in the direction orthogonal to the helical shape, when the slit patterns 85a to 85n are intermittently formed along the helical shape, girders can be automatically and easily formed. In a case in which two opposed sides extend in the direction P2 parallel to the longitudinal axis of the tubular member 83, expansion and contraction of the slitted pipe 81 in the axial direction can be prevented further.

While the girders are arranged at four positions point-symmetrical with respect to the center point of the cross section in the ninth embodiment, it is more preferable that the girders should be uniformly arranged in the entire cross section. This can further enhance torque transmissibility in a well-balanced manner.

In the ninth embodiment, the slit patterns 85a to 85n are also not formed within a distance L1 from a left end of the slitted pipe 81 and a distance L2 from a right end of the slitted pipe 81. Hence, the slitted pipe 81 is not divided by the slit patterns 85a to 85n, but can be handled as one piece. This allows the slitted pipe 81 to be handled easily.

Belt-like helical portions 87a to 87n defined by the slit patterns 85a to 85n can independently operate. Hence, for example, even if any of the helical portions 87a to 87n is cut off, the remaining helical portions can prevent the slitted pipe 81 from being cut off completely.

The slitted pipe 81 is similar to the slitted pipe 1, the slitted pipe 11, the slitted pipe 21, the slitted pipe 31, the slitted pipe 41, the slitted pipe 51, the slitted pipe 61, and the slitted pipe 71 in that, even if great force is suddenly applied to one end of the slitted pipe 81, it can be further prevented from being immediately transmitted to the other end and in that torque transmissibility to the other end of the slitted pipe 81 can be enhanced by further rotating the slitted pipe 81. Tenth Embodiment

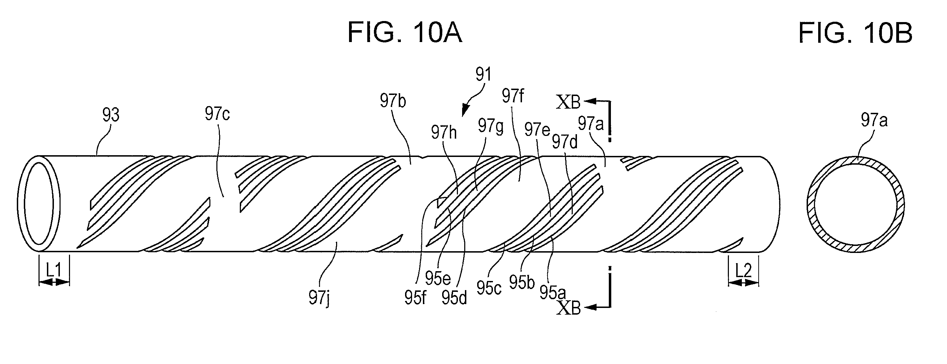

Fig. 10A is an overall perspective view of a slitted pipe according to a tenth embodiment of the present invention, and Fig. 10B is a cross-sectional view taken along line XB-XB of Fig. 10A.

Referring to Figs. 10A and 10B, a slitted pipe 91 of the tenth embodiment has six parallel and helical slit patterns 95a, 95b, 95c, 95d, 95e, and 95f extending in a longitudinal direction of a tubular member 93. Unlike the above-described first embodiment, cylindrical girders 97a, 97b, and 97c are provided at three positions except for both longitudinal end portions in the longitudinal direction of the slitted pipe 91. Within the girders, the slits of the slit pattern 95a to 95f are not formed. According to this slitted pipe 91, torque transmissibility can be further enhanced, and safety of the slitted pipe when the slitted pipe is cut off can be further improved.

In the tenth embodiment, the slit patterns 95a to 95f are also not formed within a distance L1 from a left end of the slitted pipe 91 and a distance L2 from a right end of the slitted pipe 91. Hence, the slitted pipe 91 is not divided by the slit patterns 95a to 95f, but can be handled as one piece. This allows the slitted pipe 91 to be handled easily.

Belt-like helical portions 97d to 97j defined by the slit patterns 95a to 95f can independently operate. Hence, for example, even if any of the helical portions 97d to 97j is cut off, the remaining helical portions can prevent the slitted pipe 91 from being cut off completely.

The slitted pipe 91 is similar to the slitted pipe 1, the slitted pipe 11, the slitted pipe 21, the slitted pipe 31, the slitted pipe 41, the slitted pipe 51, the slitted pipe 61, the slitted pipe 71, and the slitted pipe 81 in that, even if great force is suddenly applied to one end of the slitted pipe 91, it can be further prevented from being immediately transmitted to the other end and in that torque transmissibility to the other end of the slitted pipe 91 can be enhanced by further rotating the slitted pipe 91.

While the slitted pipes according to the embodiments of the present invention have been described above, the present invention is not limited to the above embodiments, and various modifications can be made without departing from the scope of the invention.

For example, while all of the girders are the same in the length along the helical shape in the above embodiments, they may be different according to rigidity in the longitudinal direction of the slitted pipe. In this case, the length of the girder is set to be long when rigidity of the slitted pipe is high (hard) and to be short when rigidity of the slitted pipe is low (soft).

As for the length of the girders along the helical shape, the length of the girders and the length of the slits are considered in combination. That is, rigidity of the slitted pipe in the longitudinal direction is preferably changed by setting the sum of the length of the girders and the length of the slits to be fixed (for example, L3) in all girders and changing the ratio of the length (X) along the helical shape of the girders to the length (L3-X) along the helical shape of the slits per unit length L3. According to this, it is possible to easily set a program for producing the slitted pipe and to easily produce the slitted pipe. It is also possible to easily set or change rigidity of the slitted pipe in the longitudinal direction.

The ratio of the length along the helical shape of the girders to the length of the slits along the helical shape may decrease from one end to the other end in the longitudinal direction of the slitted pipe. According to this slitted pipe, flexibility at the other end can be enhanced further.

In this case, it is also preferable to consider the length of the girders and the length of the slits in combination, as described above. Rigidity of the slitted pipe in the longitudinal direction is preferably changed by setting the sum of the length of the girders and the length of the slits to be fixed in all girders and changing the ratio of the length along the helical shape of the girders to the length along the helical shape of the slits per unit length L3. According to this, it is possible to easily set a program for producing the slitted pipe and to easily produce the slitted pipe. It is also possible to easily set or change rigidity of the slitted pipe in the longitudinal direction.

The slitted pipe may be tapered such that an outer diameter of the slitted pipe gradually decreases from one end toward the other end. According to this slitted pipe, flexibility of the slitted pipe at the other end can be enhanced further.

The outer diameter of the slitted pipe may decrease in a stepwise manner from one end toward the other end. According to this slitted pipe, flexibility of the slitted pipe at the other end can be further enhanced, and the slitted pipe can be easily produced, for example, by electropolishing.

Cases in which the slitted pipes of the above-described first to tenth embodiments are applied to guidewires will be described below with reference to the drawings.

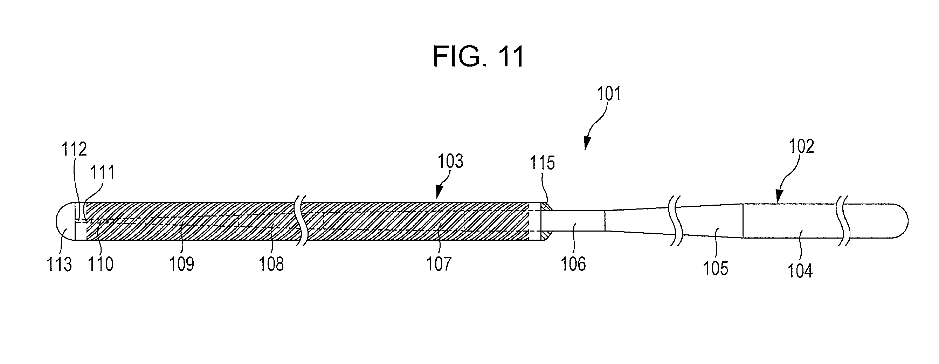

In Figs. 11 to 13, for easy understanding, the slitted pipe is schematically illustrated as a whole while being shortened in the longitudinal direction. Hence, overall dimensions of the slitted pipe are different from actual ones.

### Eleventh Embodiment

Fig. 11 is an overall view of a guidewire according to an eleventh embodiment of the present invention. A guidewire 101 of the eleventh embodiment illustrated in Fig. 11 is used, for example, for medical treatment of a blood vessel of the heart. In the eleventh embodiment, the guidewire 101 is about 1900 mm in length. The guidewire 101 includes a core shaft 102, a slitted pipe 103 according to any of the first to tenth embodiments, a distal-end brazing part 113 that joins a distal end of the core shaft 102 and a distal end of the slitted pipe 103, and a proximal-end brazing part 115 that joins the core shaft 102 and a proximal end of the slitted pipe 103.

The core shaft 102 includes a first distal end portion 112 provided at the most distal end, a second distal end portion 111 adjacent to a proximal end of the first distal end portion 112, a third distal end portion 110 adjacent to a proximal end of the second distal end portion 111, a first tapered portion 109 adjacent to a proximal end of the third distal end portion 110, a second tapered portion 108 adjacent to a proximal end of the first tapered portion 109, a third tapered portion 107 adjacent to a proximal end of the second tapered portion 108, a first cylindrical portion 106 adjacent to a proximal end of the third tapered portion 107, a fourth tapered portion 105 adjacent to a proximal end of the first cylindrical portion 106, and a cylindrical proximal end portion 104 adjacent to a proximal end of the fourth tapered portion 105.

An outer surface of a section extending from a distal end of the guidewire 101 to the fourth tapered portion 105 of the core shaft 102 via the slitted pipe 103 is coated with a hydrophilic coating agent.

While the material of the core shaft 102 is not particularly limited, stainless steel (SUS304) is used in the eleventh embodiment. As other materials, a superelastic alloy, such as a Ni-Ti alloy, and a piano wire are used.

Examples of hydrophilic materials are a cellulosic high polymer, a polyethylene oxide high polymer, a maleic anhydride high polymer (for example, a maleic anhydride copolymer such as a methyl vinyl ether-maleic anhydride copolymer), an acrylamide high polymer (for example, a block copolymer of polyacrylamide and poly(glycidyl methacrylate-dimethylacrylamide), hydrosoluble nylon, polyvinyl alcohol, polyvinylpyrrolidone, and hyaluronate. In the eleventh embodiment, hyaluronate is used as the hydrophilic material.

Since the guidewire 101 of the eleventh embodiment includes the core shaft 102 and the slitted pipe 103 covering a distal end portion of the core shaft 102, it is easy to fix the slitted pipe 103, which has no slits in both end portions, to the core shaft 102, and therefore, the guidewire 101 itself can be produced easily. Further, according to the guidewire 101 of the eleventh embodiment, torque transmissibility of the guidewire 101 in rotation in at least one direction can be enhanced, and safety of the guidewire 101 when the guidewire 101 is cut off can be improved.

In the slitted pipe according to any of the above-described fourth to seventh embodiments, the other end of the slitted pipe is preferably located on a distal side of the core shaft. In this case, the guidewire can be easily produced, torque transmissibility of the guidewire in rotation in at least one direction can be enhanced, and safety of the guidewire when the guidewire is cut off can be improved. Moreover, flexibility at the distal end portion of the guidewire can be further enhanced by the slitted pipe.

Preferably, a gap is formed between the core shaft 102 and the slitted pipe 103. In this case, flexibility can be further enhanced when the guidewire is curved, and this allows the guidewire to be easily inserted to an end portion of the blood vessel.

### Twelfth Embodiment

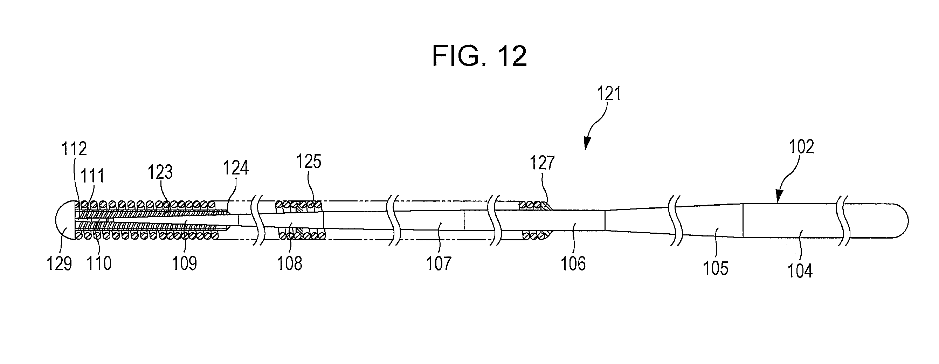

Fig. 12 is an overall view of a guidewire according to a twelfth embodiment of the present invention.

In Fig. 12, the same structures as those illustrated in Fig. 11 are denoted by the same reference numerals, and descriptions thereof are skipped.

Referring to Fig. 12, a guidewire 121 according to the twelfth embodiment includes a core shaft 102, an outer coil 125 covering a distal end portion of the core shaft 102, a slitted pipe 123 according to any of the first to tenth embodiments, which is disposed in the outer coil 125 to cover the distal end portion of the core shaft 102, a distal-end brazing part 129 that joins a distal end of the core shaft 102, a distal end of the outer coil 125, and a distal end of the slitted pipe 123, an inner proximal-end brazing part 124 that joins a proximal end of the slitted pipe 123 and the core shaft 102, and a proximal-end brazing part 127 that joins the core shaft 102 and a proximal end of the outer coil 125.

An outer surface of a section extending from a distal end of the guidewire 121 to a fourth tapered portion 105 of the core shaft 102 via the outer coil 125 is coated with a hydrophilic coating agent.

The guidewire 121 of the twelfth embodiment includes the core shaft 102, the outer coil 125 covering the distal end portion of the core shaft 102, and the slitted pipe 123 disposed in the outer coil 125 to cover the distal end portion of the core shaft 102. Hence, the guidewire 121 can be easily produced, torque transmissibility of the guidewire 121 in rotation in at least one direction can be enhanced, and safety of the guidewire 121 when the guidewire 121 is cut off can be improved. Moreover, flexibility of the guidewire 121 can be adjusted by the slitted pipe 123 disposed in the outer coil 125 while the outer diameter of the outer coil 125 is maintained.

In the slitted pipe according to any of the fourth to seventh embodiments, the other end of the slitted pipe is preferably located on a distal side of the core shaft. In this case, the guidewire 121 can be easily produced, torque transmissibility of the guidewire 121 in rotation in at least one direction can be enhanced, and safety of the guidewire 121 when the guidewire 121 is cut off can be improved. Moreover, flexibility at the distal end of the guidewire 121 can be further enhanced by the slitted pipe 123 disposed in the outer coil 125 while the outer diameter of the outer coil 125 is maintained.

Preferably, gaps are formed between the core shaft 102 and the slitted pipe 123 and between the outer coil 125 and the slitted pipe 123. In this case, flexibility can be enhanced when the guidewire 121 is curved, and this allows the guidewire 121 to be easily inserted to an end portion of the blood vessel.

### Thirteenth Embodiment

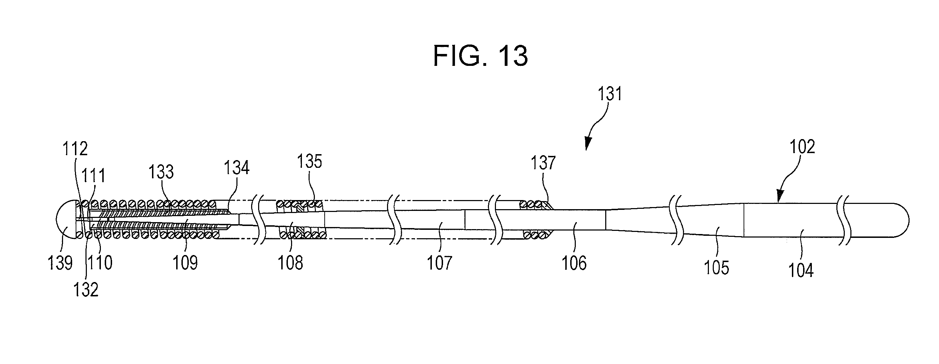

Fig. 13 is an overall view of a guidewire according to a thirteen the embodiment of the present invention.

In Fig. 13, the same structures as those illustrated in Fig. 11 are denoted by the same reference numerals, and descriptions thereof are skipped.

Referring to Fig. 13, a guidewire 131 of the thirteenth embodiment includes a core shaft 102, an outer coil 135 covering a distal end portion of the core shaft 102, a slitted pipe 133 according to any of the first to tenth embodiments, which is disposed in the outer coil 135 to cover the distal end portion of the core shaft 102, a distal-end brazing part 139 that joins a distal end of the core shaft 102 and a distal end of the outer coil 135, an inner distal-end brazing part 132 that joins a distal end of the slitted pipe 133 and the core shaft 102, an inner proximal-end brazing part 134 that joins a proximal end of the slitted pipe 133 and the core shaft 102, and a proximal-end brazing part 137 that joins the core shaft 102 and a proximal end of the outer coil 135.

In the thirteenth embodiment, the distal end of the slitted pipe 133 is located a predetermined distance apart from the distal end of the core shaft 102 toward the proximal end. Further, an outer surface of a section extending from a distal end of the guidewire 131 to a fourth tapered portion 105 of the core shaft 102 via the outer coil 135 is coated with a hydrophilic coating agent.

According to the guidewire 131 of the thirteenth embodiment, since the distal end of the slitted pipe 133 is located a predetermined distance apart from the distal end of the core shaft 102 toward the proximal end, the distal end portion of the guidewire 131 can be easily bent, and vascular selectivity of the guidewire 131 can be enhanced.

Preferably, gaps are provided between the core shaft 102 and the slitted pipe 133 and between the outer coil 135 and the slitted pipe 133. In this case, flexibility can be enhanced when the guidewire 131 is curved, and this allows the guidewire 131 to be easily inserted to an end portion of the blood vessel.

### Eighteenth Aspect

In the slitted pipe of the eight aspect, a length of the girders along the helical shape is changed according to rigidity in the longitudinal direction.

### Nineteenth Aspect

In the slitted pipe of the eighteenth aspect, a ratio of the length of the girders along the helical shape to a length of the slits along the helical shape per unit length is changed according to the rigidity in the longitudinal direction.

### Twentieth Aspect

In the slitted pipe of the nineteenth aspect, the ratio of the length of the girders along the helical shape to the length of the slits along the helical shape per unit length decreases from one end toward the other end in the longitudinal direction.

## Claims

1. A slitted pipe (1; 11; 21; 31; 41; 51; 61; 71; 81; 91) comprising a plurality of parallel slit patterns (5a to 5c; 15a to 15f; 25a to 25n; 35a to 35n; 45a to 45n; 55a to 55n; 65a to 65n; 75a to 75n; 81a to 85n; 95a to 95f) provided in an area except for both end portions in a longitudinal direction to extend in the longitudinal direction in a helical shape.

2. The slitted pipe according to Claim 1, wherein the helical slit patterns (5a to 5f) are arranged at positions point-symmetrical with respect to a center point of a cross section.

3. The slitted pipe according to Claim 1, wherein the helical slit patterns (25a to 25n) are uniformly arranged in the entire cross section.

4. The slitted pipe according to any one of Claims 1 to 3, wherein a pitch of the helical slit patterns (35a to 35n) gradually decreases from one end toward the other end.

5. The slitted pipe according to any one of Claims 1 to 3, wherein a pitch of the helical slit patterns (45a to 45n) decreases in a stepwise manner from one end toward the other end.

6. The slitted pipe according to any one of Claims 1 to 5, wherein a width of the helical slit patterns (55a to 55n) gradually increases from one end toward the other end.

7. The slitted pipe according to any one of Claims 1 to 5, wherein a width of the helical slit patterns (65a to 65n) increases in a stepwise manner from one end toward the other end.

8. The slitted pipe according to any one of Claims 1 to 7, wherein girders (79a to 79g) are provided on the helical slit pattern (75a to 75n) to divide the slit pattern (75a to 75n) into a plurality of slits.

9. The slitted pipe according to Claim 8, wherein girders (79a to 79g) adjacent in the longitudinal direction are arranged while being shifted from each other by a predetermined angle in cross section per predetermined distance in the longitudinal direction.

10. The slitted pipe according to Claim 8 or 9, wherein the girders (89a to 89f) provided on the slit patterns form at least one helical shape different from the helical shape of the slit patterns.

11. The slitted pipe according to any one of Claims 8 to 10, wherein the girders (97a to 97c) are provided in a cylindrical form at at least one position other than the end portions in the longitudinal direction.

12. The slitted pipe according to any one of Claims 1 to 11, wherein the slitted pipe is circular in cross section.

13. A guidewire (101; 121; 131 comprising:
a core shaft (102); and
a slitted pipe (1, 11, 21, 31, 41, 51, 61, 71, 81, 91) according to any one of Claims 1 to 12, the slitted pipe (1, 11, 21, 31, 41, 51, 61, 71, 81, 91) covering a distal end portion of the core shaft (102).

14. The guidewire according to Claim 13 comprising:
a slitted pipe (31, 41, 51, 61) according to any one of Claims 4 to 7, the slitted pipe (31, 41, 51, 61) covering the distal end portion of the core shaft (102) such that the other end of the slitted pipe (31, 41, 51, 61) is located on a distal side of the core shaft (102).

15. The guidewire according to Claim 13, further comprising:
a coil body (125) covering a distal end portion of the core shaft (102);
wherein the slitted pipe (1, 11, 21, 31, 41, 51, 61, 71, 81, 91) is disposed in the coil body (125) to cover the distal end portion of the core shaft (102).

16. The guidewire according to Claim 13 comprising:
a slitted pipe (31, 41, 51, 61) according to any one of Claims 4 to 7, wherein the slitted pipe (31, 41, 51, 61) is disposed in the coil body (125) to cover the distal end portion of the core shaft (102) such that the other end of the slitted pipe (31, 41, 51, 61) is located on a distal side of the core shaft (102).

17. The guidewire according to Claim 15 or 16, wherein a distal end of the slitted pipe (133) is located a predetermined distance apart from a distal end toward a proximal end of the core shaft (102).
